(19) Europäisches Patentamt
European Patent Office
Office européen des brevets

(11) Veröffentlichungsnummer: **0 252 286 B1**

(12) **EUROPÄISCHE PATENTSCHRIFT**

(45) Veröffentlichungstag der Patentschrift: **08.07.92**

(51) Int. Cl.⁵: **A61K 31/165**

(21) Anmeldenummer: **87107958.8**

(22) Anmeldetag: **02.06.87**

# (54) Neue pharmazeutische Präparate zur topischen Applikation, Verfahren zu ihrer Herstellung und ihre Verwendung.

(30) Priorität: **05.06.86 CH 2290/86**

(43) Veröffentlichungstag der Anmeldung:
**13.01.88 Patentblatt 88/02**

(45) Bekanntmachung des Hinweises auf die Patenterteilung:
**08.07.92 Patentblatt 92/28**

(84) Benannte Vertragsstaaten:
**BE CH DE FR GB IT LI LU NL SE**

(56) Entgegenhaltungen:

**AGENTS AND ACTIONS, vol. 19, no. 5/6, 18.-20. Juni 1986, Seiten 368-370, Birkhäuser Verlag, Basel, CH; W. PIGNAT et al.: "How specific is the arachidonic acid-induced mouse ear oedema for lipoxygenase (LO)- and cyclooxygenase (CO)- inhibitors?**

(73) Patentinhaber: **CIBA-GEIGY AG**
**Klybeckstrasse 141**
**CH-4002 Basel(CH)**

(72) Erfinder: **Böttcher, Irmgard, Dr.**
**Frobenstrasse 46**
**CH-4053 Basel(CH)**
Erfinder: **Pignat, Werner**
**Erlenweg 4**
**CH-5200 Windisch(CH)**

(74) Vertreter: **Zumstein, Fritz, Dr. et al**
**Bräuhausstrasse 4**
**W-8000 München 2(DE)**

**EP 0 252 286 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue pharmazeutische Präparate zur topischen, d.h. dermalen, Applikation, die als Wirkstoff eine Verbindung der Formel I

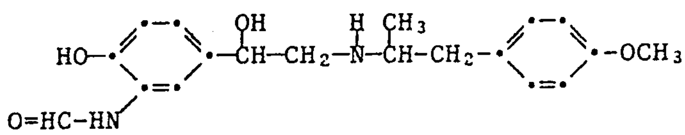 (I),

insbesondere 2-Hydroxy-5-[(RS)-1-hydroxy-2-[[(RS)-1-(p-methoxyphenyl)-prop-2-yl]-amino]-äthyl]-formanilid (Formoterol), oder eines ihrer pharmazeutisch verwendbaren Salze, insbesondere ihr Semifumarat, enthalten, Verfahren zur Herstellung solcher pharmazeutischer Präparate und die Verwendung von Verbindungen der Formel I und ihrer pharmazeutisch verwendbaren Salze.

Pharmazeutisch verwendbare Salze einer Verbindung der Formel I sind pharmazeutisch verwendbare Säureadditionssalze, beispielsweise Salze mit anorganischen Säuren, wie Mineralsäuren, mit Sulfaminsäuren, wie Cyclohexylsulfaminsäure, mit organischen Carbonsäuren, wie Niederalkancarbonsäuren oder gegebenenfalls ungesättigten Dicarbonsäuren, mit durch Hydroxy und/oder Oxo substituierten aliphatischen Carbonsäuren oder mit aliphatischen oder aromatischen Sulfonsäuren, beispielsweise Sulfate oder Hydrohalogenide, wie Hydrobromide oder Hydrochloride, Oxalate, Malonate, Fumarate oder Maleinate, Tartrate, Pyruvate oder Citrate sowie Sulfonate, wie Methan-, Benzol- oder p-Toluolsulfonate. Dabei sind unter Niederalkancarbonsäuren vorzugsweise solche zu verstehen, die im Niederalkylteil bis und mit 7, vor allem bis und mit 4 Kohlenstoffatome (C-Atome) enthalten.

Die pharmazeutisch verwendbaren Salze einer Verbindung der Formel I können auch in der Form ihrer Hydrate vorliegen oder andere, beispielsweise zur Kristallisation verwendete, pharmazeutisch unbedenkliche Lösungsmittel einschliessen.

Infolge der engen Beziehung zwischen einer Verbindung der Formel I in freier Form und in Form ihrer pharmazeutisch verwendbaren Salze sind vorstehend und nachfolgend unter einer freien Verbindung oder ihren Salzen sinn- und zweckgemäss gegebenenfalls auch die entsprechenden Salze bzw. die freie Verbindung zu verstehen. Nachstehend sind dementsprechend unter Formoterol sowohl die freie Base als auch seine pharmazeutisch verwendbaren Salze, insbesondere sein Semifumarat, zu verstehen.

Formoterol und seine pharmazeutisch verwendbaren Salze sind bekannt und beispielsweise in der deutschen Offenlegungsschrift DE-2 305 092 (Yamanouchi Pharmaceutical Co. Ltd., Tokio) beschrieben.

Wie dort dargelegt, gehören Formoterol und seine pharmazeutisch verwendbaren Salze zur Klasse der $\beta$-adrenergen Stimulatoren und sind angabegemäss aufgrund ihrer grossen Aktivität auf die glatte Atmungsmuskulatur als bronchodilatatorische Mittel geeignet, wobei orale und parenterale Darreichungsformen beschrieben werden.

Pharmazeutische Präparate von Verbindungen der Formel I beziehungsweise ihren pharmazeutisch verwendbaren Salzen zur topischen, d.h. dermalen, Applikation auf die Haut und/oder Schleimhaut sind dagegen neu. Im Stand der Technik finden sich keinerlei diesbezügliche Hinweise.

Im Rahmen der Erfindung zeigte sich nun überraschenderweise, dass insbesondere Formoterol als zusätzliche wertvolle pharmakologische Eigenschaft bei topischer, d.h. dermaler, Applikation auf die Haut und/oder Schleimhaut eine stark ausgeprägte antiphlogistische (hautphlogistatische), d.h. topisch-antiinflammatorische, Wirkung aufweist.

Vorstehend und nachfolgend sind unter pharmazeutischen Präparaten zur topischen, d.h. dermalen, Applikation auf die Haut und/oder Schleimhaut dermatologische pharmazeutische Präparate zur äusserlichen Anwendung auf der äusseren Haut, einschliesslich der Augenbindehaut, der Lippen und der Genital- und Analregion, zu verstehen.

Die antiphlogistische Wirkung von Formoterol und seinen pharmazeutisch verwendbaren Salzen lässt sich an geeigneten Tiermodellen zeigen. So ergaben Untersuchungen mit Formoterol in Form der freien Base im Konzentrationsbereich von etwa 0,03 bis etwa 30 mg/ml am durch Crotonöl induzierten experimentellen Ohroedem der Ratte eine Hemmkonzentration $EC_{50}$ von etwa 0,5 mg/ml und im Konzentrationsbereich von etwa 0,01 bis etwa 10 mg/ml am durch Crotonöl induzierten experimentellen Ohroedem der Maus eine Hemmkonzentration $EC_{50}$ von etwa 0,32 mg/ml [Methodik: in Anlehnung an G. Tonelli et al., Endocrinology 77, 625 (1965)].

Eine stark ausgeprägte antiphlogistische Wirkung lässt sich auch am durch Arachidonsäure induzierten experimentellen Ohroedem der Maus nachweisen. In diesem Modell ergab sich für Formoterol in Form der

freien Base im Konzentrationsbereich von etwa 0,0005 bis etwa 50 mg/ml eine Hemmkonzentration $EC_{50}$ von etwa 0,0026 mg/ml [Methodik: in Anlehnung an J.M. Young et al, J.Invest.Dermatol. 82, 367 (1984)].

Formoterol ist daher vorzüglich als dermatologisches Antiphlogistikum zur Behandlung von entzündlichen oder proliferativen, mit Entzündung verbundenen Dermatosen geeignet, insbesondere in Form der erfindungsgemässen topisch applizierbaren dermatologischen pharmazeutischen Präparate. Es kann bei verschiedenartigsten entzündlichen Dermatosen, insbesondere solchen akuter und subchronischer Art, sowohl allergischer als auch nicht-allergischer Natur, Verwendung finden. Weiterhin ist Formoterol zur Behandlung von proliferativen Hauterkrankungen, insbesondere solchen, die mit Entzündung verbunden sind, wie der Psoriasis, geeignet, und kann ebenso zur Behandlung von Hautirritationen, Exanthemen und Verbrennungen sowie zur Behandlung von Entzündungen der Augenbindehaut, der Lippen und der Genital- und Analregion Verwendung finden.

Bei den erfindungsgemässen pharmazeutischen Präparaten, welche eine Verbindung der Formel I, insbesondere Formoterol, oder pharmazeutisch verwendbare Salze davon enthalten, handelt es sich um solche zur dermatologischen Anwendung an Warmblütern, wobei der pharmakologische Wirkstoff allein oder zusammen mit einem pharmazeutisch anwendbaren Trägermaterial enthalten ist. Die tägliche Dosierung des Wirkstoffs hängt von dem Alter und dem individuellen Zustand sowie von der Applikationsweise ab. Entsprechende Mittel mit einem Wirkstoffgehalt von etwa 0,00001 bis etwa 1 Gewichtsprozent, z.B. in Form von Crèmes, Salben oder Lösungen, können beispielsweise 2 bis 3 x täglich appliziert werden.

Als dermatologisch anwendbare pharmazeutische Präparate kommen in erster Linie Crèmes, Salben, Fettsalben, Pasten, Gele, Schäume, Tinkturen und Lösungen sowie zur Behandlung der Augenbindehaut Augentropfen in Frage, die jeweils beispielsweise von etwa 0,00001 bis etwa 1 Gewichtsprozent, insbesondere von etwa 0,0005 bis etwa 0,5 Gewichtsprozent, des Wirkstoffs enthalten.

Crèmes sind Öl-in-Wasser Emulsionen, die mehr als 50 % Wasser aufweisen. Als ölige Grundlage verwendet man in erster Linie Fettalkohole, z.B. Lauryl-, Cetyl- oder Stearylalkohol, Fettsäuren, z.B. Palmitin- oder Stearinsäure, flüssige bis feste Wachse, z.B. Isopropylmyristat, Wollwachs oder Bienenwachs, und/oder Kohlenwasserstoffe, z.B. Vaseline (Petrolatum) oder Paraffinöl. Als Emulgatoren kommen oberflächenaktive Substanzen mit vorwiegend hydrophilen Eigenschaften in Frage, wie entsprechende nichtionische Emulgatoren, z.B. Fettsäureester von Polyalkoholen oder Äthylenoxidaddukte davon, wie Polyglycerinfettsäureester oder Polyoxyäthylensorbitanfettsäureester (Tweens®), ferner Polyoxyäthylenfettalkoholäther oder -fettsäureester, oder entsprechende, ionische Emulgatoren, wie Alkalimetallsalze von Fettalkoholsulfaten, z.B. Natriumlaurylsulfat, Natriumcetylsulfat oder Natriumstearylsulfat, die man üblicherweise in Gegenwart von Fettalkoholen, z.B. Cetylalkohol oder Stearylalkohol, verwendet. Zusätze zur Wasserphase sind u.a. Mittel, welche die Austrocknung der Crème vermindern, z.B. Polyalkohole, wie Glycerin, Sorbit, Propylenglykol und/oder Polyäthylenglykole, ferner Konservierungsmittel, Riechstoffe, etc.

Salben sind Wasser-in-Öl-Emulsionen, die bis zu 70 %, vorzugsweise jedoch von etwa 20 % bis etwa 50 % Wasser oder wässrige Phasen enthalten. Als Fettphase kommen in erster Linie Kohlenwasserstoffe, z.B. Vaseline, Paraffinöl und/oder Hartparaffine in Frage, die zur Verbesserung des Wasserbindungsvermögens vorzugsweise geeignete Hydroxyverbindungen, wie Fettalkohole oder Ester davon, z.B. Cetylalkohol oder Wollwachsalkohole bzw. Wollwachs, enthalten. Emulgatoren sind entsprechende lipophile Substanzen, wie Sorbitanfettsäureester (Spans®), z.B. Sorbitanoleat und/oder Sorbitanisostearat. Zusätze zur Wasserphase sind u.a. Feuchthaltungsmittel, wie Polyalkohole, z.B. Glycerin, Propylenglykol, Sorbit und/oder Polyäthylenglykol, sowie Konservierungsmittel, Riechstoffe, etc.

Fettsalben sind wasserfrei und enthalten als Grundlage insbesondere Kohlenwasserstoffe, z.B. Paraffin, Vaseline und/oder flüssige Paraffine, ferner natürliche oder partialsynthetische Fette, z.B. Kokosfettsäuretriglycerid, oder vorzugsweise gehärtete Öle, z.B. hydriertes Erdnuss- oder Rizinusöl, ferner Fettsäurepartialester des Glycerins, z.B. Glycerinmono- und -distearat, sowie z.B. die im Zusammenhang mit den Salben erwähnten, die Wasseraufnahmefähigkeit steigernden Fettalkohole, Emulgatoren und/oder Zusätze.

Pasten sind Crèmes und Salben mit sekretabsorbierenden Puderbestandteilen, wie Metalloxiden, z.B. Titanoxid oder Zinkoxid, ferner Talk und/oder Aluminiumsilikate, welche die Aufgabe haben, vorhandene Feuchtigkeit oder Sekrete zu binden.

Bei den Gelen wird zwischen wässrigen und wasserfreien oder wasserarmen Gelen unterschieden, die aus quellbaren, gelbildenden Materialien bestehen. In erster Linie kommen transparente Hydrogele auf der Grundlage anorganischer oder organischer Makromoleküle zum Einsatz. Hochmolekulare anorganische Komponenten mit gelbildenden Eigenschaften sind überwiegend wasserhaltige Silikate, wie Aluminiumsilikate, z.B. Betonit, Magnesium-Aluminium-Silikate, z.B. Veegum, oder kolloidale Kieselsäure, z.B. Aerosil®. Als hochmolekulare organische Stoffe werden beispielsweise natürliche, halbsynthetische oder synthetische Makromoleküle verwendet. Natürliche und halbsynthetische Polymere leiten sich beispielsweise von Polysacchariden mit den unterschiedlichsten Kohlenhydratbausteinen ab, wie Cellulosen, Stärken, Tragant,

arabisches Gummi, Agar-Agar, Gelatine, Alginsäure und deren Salze, z.B. Natriumalginat, und deren Derivate, wie Niederalkylcellulosen, z.B. Methyl-oder Äthylcellulosen, Carboxy- oder Hydroxyniederalkylcellulose, z.B. Carboxymethyl- oder Hydroxyäthylcellulosen. Die Bausteine synthetischer, gelbildender Makromoleküle sind beispielsweise entsprechend substituierte ungesättigte Aliphaten, wie Vinylalkohol, Vinylpyrrolidin, Acryl- oder Methacrylsäure. Als Beispiele für solche Polymere sind Polyvinylalkohol-Derivate, wie Polyviol, Polyvinylpyrrolidine, wie Kollidon®, Polyacrylate bzw. Polymethacrylate, wie Rohagit® S oder Eudispert®, zu nennen. Den Gelen können übliche Zusatzmittel, wie Konservierungs- oder Riechstoffe, zugegeben werden.

Schäume werden z.B. aus Druckbehältern verabreicht und sind in Aerosolform vorliegende flüssige Öl-in-Wasser-Emulsionen, wobei halogenierte Kohlenwasserstoffe, wie Chlorfluorniederalkane, z.B. Dichlordifluormethan und Dichlortetrafluoräthan, als Treibmittel verwendet werden. Als Ölphase verwendet man u.a. Kohlenwasserstoffe, z.B. Paraffinöl, Fettalkohole, z.B. Cetylalkohol, Fettsäureester, z.B. Isopropylmyristat, und/oder andere Wachse. Als Emulgatoren verwendet man u.a. Gemische von solchen mit vorwiegend hydrophilen Eigenschaften, wie Polyoxyäthylen-sorbitan-fettsäureester (Tweens®), und solchen mit vorwiegend lipophilen Eigenschaften, wie Sorbitanfettsäureester (Spans®). Dazu kommen die üblichen Zusätze, wie Konservierungsmittel, etc.

Tinkturen und Lösungen weisen meistens eine wässrig-äthanolische Grundlage auf, der u.a. Polyalkohole, z.B. Glycerin, Glykole und/oder Polyäthylenglykol, als Feuchthaltemittel zur Herabsetzung der Verdunstung, und rückfettende Substanzen, wie Fettsäureester mit niedrigen Polyäthylenglykolen, d.h. im wässrigen Gemisch lösliche, lipophile Substanzen als Ersatz für die der Haut mit dem Äthanol entzogenen Fettsubstanzen, und, falls notwendig, andere Hilfs- und Zusatzmittel beigegeben sind.

Augentropfen sind zumeist sterile wässrige Lösungen, die auf den physiologischen pH-Bereich von 6 bis 8 eingestellt sind. Die Konzentration der zur pH-Einstellung beispielsweise verwendeten Puffer, wie Phosphat- oder Acetatpuffer, ist so gewählt, dass der pH-Wert der Tränenflüssigkeit nicht langfristig beeinflusst und dadurch eine Schmerzempfindung ausgelöst wird. Die Isotonie mit der Tränenflüssigkeit wird meist durch Zugabe von Salzen, wie Natriumcitrat oder vorzugsweise Natriumchlorid, oder Mannit erreicht. Zusätzlich werden die üblichen Hilfsstoffe, beispielsweise Antioxidantien, wie Natriumpyrosulfit, Konservierungsmittel, wie Benzalkoniumchlorid, Cetylpyridiniumchlorid oder 2-Phenyl-äthylalkohol, sowie gegebenenfalls Lösungsvermittler, z.B. Polyoxyäthylensorbitanmonooleat, Polyoxyäthylenglykol oder $\alpha$- bzw. $\beta$-Cyclodextrin, zugesetzt.

Die Herstellung der dermatologisch anwendbaren pharmazeutischen Präparate erfolgt in an sich bekannter Weise durch Vermischung mit dafür üblichen pharmazeutischen Hilfsstoffen, z.B. durch Lösen oder Suspendieren des Wirkstoffs in der Grundlage oder in einem Teil davon, falls notwendig. Zur Herstellung von Emulsionen, in denen der Wirkstoff in einer der flüssigen Phasen gelöst ist, wird der Wirkstoff in der Regel vor der Emulgierung in dieser gelöst; zur Herstellung von Suspensionen, in denen der Wirkstoff in der Emulsion suspendiert ist, wird der Wirkstoff nach der Emulgierung mit einem Teil der Grundlage vermischt und dann dem Rest der Formulierung beigegeben.

Die nachfolgenden Beispiele illustrieren die oben beschriebene Erfindung, sie sollen jedoch diese in ihrem Umfang in keiner Weise einschränken. Temperaturen sind in Celsiusgraden angegeben.

Beispiel 1: Eine Salbe, enthaltend 0,05 % 2-Hydroxy-5-[(RS)-1-hydroxy-2-[[(RS)-1-(p-methoxyphenyl)-prop-2-yl]-amino]-äthyl]-formanilid oder sein Semifumarat, kann wie folgt hergestellt werden:

| Zusammensetzung: | |
|---|---|
| Wirkstoff | 0,05 % |
| Vaseline | 45,00 % |
| Paraffinöl | 19,60 % |
| Cetylalkohol | 5,00 % |
| Bienenwachs | 5,00 % |
| Sorbitan-sesquioleat | 5,00 % |
| p-Hydroxybenzoesäureester | 0,20 % |
| Wasser, entmineralisiert, bis zu | 100,00 % |

Die Fettstoffe und Emulgatoren werden zusammengeschmolzen. Das Konservierungsmittel wird in Wasser gelöst, und die Lösung in die Fettschmelze bei erhöhter Temperatur einemulgiert. Nach dem Erkalten wird eine Suspension des Wirkstoffs in einem Teil der Fettschmelze in die Emulsion eingearbeitet.

Beispiel 2: Eine Crème, enthaltend 0,5 % 2-Hydroxy-5-[(RS)-1-hydroxy-2-[[(RS)-1-(p-methoxyphenyl)-prop-2-yl]-amino]-äthyl]-formanilid oder sein Semifumarat, kann wie folgt hergestellt werden:

| Zusammensetzung: | |
|---|---|
| Wirkstoff | 0,5 % |
| Isopropylpalmitat | 8,0 % |
| Cetylpalmitat | 1,5 % |
| Siliconöl 100 | 0,5 % |
| Sorbitanmonostearat | 3,0 % |
| Polysorbat 60 | 3,5 % |
| 1,2-Propylenglykol PH | 20,0 % |
| Acrylsäurepolymerisat | 0,5 % |
| Triäthanolamin | 0,7 % |
| Wasser, entmineralisiert, bis zu | 100,0 % |

Das Acrylsäurepolymerisat wird in einem Gemisch aus entmineralisiertem Wasser und 1,2-Propylenglykol suspendiert. Unter Rühren wird hierauf Triäthanolamin zugegeben, wodurch ein Schleim erhalten wird. Ein Gemisch aus Isopropylpalmitat, Cetylpalmitat, Siliconöl, Sorbitanmonostearat und Polysorbat wird auf ca. 75° erwärmt und unter Rühren in den gleichfalls auf ca. 75° erwärmten Schleim eingearbeitet. Die auf Raumtemperatur abgekühlte Crème-Grundlage wird hierauf zur Herstellung eines Konzentrates mit dem Wirkstoff verwendet. Das Konzentrat wird mittels eines Durchlaufhomogenisators homogenisiert und dann portionsweise der Grundlage hinzugefügt.

Beispiel 3: Eine Crème, enthaltend 0,05 % 2-Hydroxy-5-[(RS)-1-hydroxy-2-[[(RS)-1-(p-methoxyphenyl)-prop-2-yl]-amino-äthyl]-formanilid oder sein Semifumarat, kann wie folgt erhalten werden:

| Zusammensetzung: | |
|---|---|
| Wirkstoff | 0,05 % |
| Cetylpalmitat PH | 2,00 % |
| Cetylalkohol PH | 2,00 % |
| Triglyceridgemisch gesättigter mittelfettiger Fettsäuren | 5,00 % |
| Stearinsäure | 3,00 % |
| Glycerinstearat PH | 4,00 % |
| Cetomacrogol 1000 | 1,00 % |
| mikrokristalline Cellulose | 0,50 % |
| 1,2-Propylenglykol, dest. | 20,00 % |
| Wasser, entmineralisiert, bis zu | 100,00 % |

Cetylalkohol, Cetylpalmitat, das Triglyceridgemisch, Stearinsäure und Glycerinstearat werden zusammengeschmolzen. Die mikrokristalline Cellulose wird in einem Teil des Wassers dispergiert. Im restlichen Teil des Wassers wird Cetomacrogol gelöst und das Propylenglykol sowie der Schleim dazu gemischt. Die Fettphase wird anschliessend unter Rühren zur Wasserphase gegeben und kaltgerührt. Schliesslich wird der Wirkstoff mit einem Teil der Grundlage angerieben und hierauf die Anreibung in den Rest der Crème eingearbeitet.

Beispiel 4: Ein transparentes Hydrogel, enthaltend 0,5 % 2-Hydroxy-5-[(RS)-1-hydroxy-2-[[(RS)-1-(p-methoxyphenyl)-prop-2-yl]amino]-äthyl]-formanilid oder sein Semifumarat, wird wie folgt hergestellt:

| Zusammensetzung: | |
|---|---|
| Wirkstoff | 0,5 % |
| Propylenglykol | 10,0 - 20,0 % |
| Isopropanol | 20,0 % |
| Hydroxypropyl-methylcellulose | 2,0 % |
| Wasser | ad 100,0 % |

Die Hydroxypropyl-methylcellulose wird im Wasser gequollen. Der Wirkstoff wird in einem Gemisch aus Isopropanol und Propylenglykol gelöst. Anschliessend wird die Wirkstofflösung mit einem gequollenen Cellulose-Derivat vermischt und, wenn erwünscht, mit Riechstoffen (0,1 %) versetzt.

Beispiel 5: Ein transparentes Hydrogel, enthaltend 0,005 % 2-Hydroxy-5-[(RS)-1-hydroxy-2-[[(RS)-1-(p-methoxyphenyl)-prop-2-yl]-amino]-äthyl]-formanilid oder sein Semifumarat, wird wie folgt hergestellt:

| Zusammensetzung: | |
|---|---|
| Wirkstoff | 0,005 % |
| Propylenglykol | 20,0 % |
| Isopropanol | 20,0 % |
| Acrylsäurepolymerisat | 2,0 % |
| Triäthanolamin | 3,0 % |
| Wasser | ad 100,0 % |

Acrylsäurepolymerisat und Wasser werden dispergiert und mit Triäthanolamin neutralisiert. Der Wirkstoff wird in einem Gemisch aus Isopropanol und Propylenglykol gelöst. Anschliessend wird die Wirkstofflösung mit dem Gel vermischt, wobei, wenn erwünscht, Riechstoff (0,1 %) zugegeben werden kann.

Beispiel 6: Ein Schaumspray, enthaltend 0,01 % 2-Hydroxy-5-[(RS)-1-hydroxy-2-[[(RS)-1-(p-methoxyphenyl)-prop-2-yl]-amino]-äthyl]-formanilid oder sein Semifumarat, kann folgendermassen hergestellt werden:

| Zusammensetzung: | |
|---|---|
| Wirkstoff | 0,01 % |
| Cetylalkohol PH | 1,70 % |
| Paraffinöl, dickflüssig | 1,00 % |
| Isopropylmyristat | 2,00 % |
| Cetomacrogol 1000 | 2,40 % |
| Sorbitanmonostearat | 1,50 % |
| 1,2-Propylenglykol PH | 5,00 % |
| Methylparaben | 0,18 % |
| Propylparaben | 0,02 % |
| Chemoderm® 314 | 0,10 % |
| Wasser, entmineralisiert, bis zu | 100,00 % |

Cetylalkohol, Paraffinöl, Isopropylmyristat, Cetomacrogol und Sorbitanstearat werden zusammengeschmolzen. Methyl- und Propylparaben werden in heissem Wasser gelöst. Die Schmelze und die Lösung werden anschliessend vermischt. Der Wirkstoff, in Propylenglykol suspendiert, wird in die Grundlage eingearbeitet. Anschliessend wird Chemoderm® zugeführt und mit Wasser auf das Endgewicht ergänzt.

Abfüllung:

20 ml des Gemisches werden in eine Aluminiumblockdose eingefüllt. Die Dose wird mit einem Ventil versehen, und das Treibgas wird unter Druck eingefüllt.

Beispiel 7: Augentropfen, enthaltend 0,3 % 2-Hydroxy-5-[(RS)-1-hydroxy-2-[[(RS)-1-(p-methoxyphenyl)-prop-2-yl]-amino]-äthyl]-formanilid oder sein Semifumarat, können z.B. folgendermassen hergestellt werden:

| Zusammensetzung (für 10 000 Flaschen mit je 10 ml Augentropfenlösung): | |
|---|---|
| Wirkstoff | 0,30 % |
| Dinatriumphosphat | 0,31 % |
| Citronensäure | 0,15 % |
| Natriumchlorid | 0,35 % |
| Natriumpyrosulfit | 0,10 % |
| Benzalkoniumchlorid | 0,01 % |
| Wasser, entmineralisiert, bis zu | 100,00 % |

Der Wirkstoff und alle angegebenen Zusatzsstoffe werden unter einer Stickstoffatmosphäre in 80 l entmineralisertes Wasser eingerührt. Nach der vollständigen Auflösung aller Bestandteile wird die Lösung mit entmineralisertem Wasser auf 100 l aufgefüllt, in einem Autoklav bei 120° während 20 Minuten sterilisiert und anschliessend unter sterilen Bedingungen durch ein Membranfilter (Porendurchmesser: 0,2 µm) filtriert. Je 10 ml des Filtrats werden unter aseptischen Bedingungen in eine Flasche mit Tropfpipettenverschluss abgefüllt.

In analoger Weise wie in den Beispielen 1 bis 7 beschrieben kann man auch dermatologisch anwendbare pharmazeutische Präparate, die ein anderes pharmazeutisch verwendbares Salz von Formoterol enthalten, herstellen.

Ebenso kann man auch dermatologisch anwendbare pharmazeutische Präparate, die eine andere Verbindung der Formel I oder ein pharmazeutisch verwendbares Salz davon enthalten, herstellen.

**Patentansprüche**

1. Pharmazeutisches Präparat, enthaltend eine Verbindung der Formel I

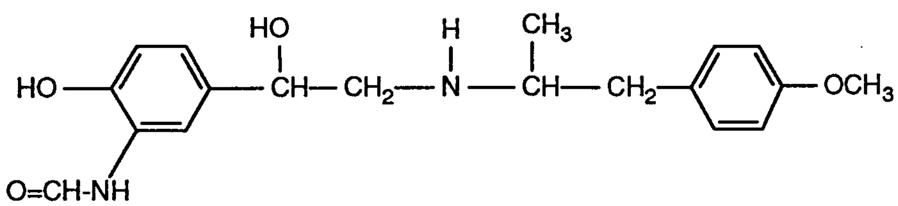

(I)

oder ein pharmazeutisch verwendbares Salze davon neben üblichen pharmazeutischen Hilfsstoffen, dadurch gekennzeichnet, dass es ausschliesslich für die topische, d.h. dermale, Applikation bestimmt ist.

2. Pharmazeutisches Präparat gemäss Anspruch 1, enthaltend als Wirkstoff das Semifumarat einer Verbindung der Formel I.

3. Pharmazeutisches Präparat gemäss Anspruch 1 oder 2, enthaltend einen Wirkstoffanteil von etwa 0,00001 bis etwa 1 Gewichtsprozent.

4. Pharmazeutisches Präparat gemäss Anspruch 3, enthaltend einen Wirkstoffanteil von etwa 0,00005 bis etwa 0,5 Gewichtsprozent.

5. Pharmazeutisches Präparat gemäss einem der Ansprüche 1 bis 4 in Form einer Crème, einer Salbe, einer Fettsalbe, einer Paste, eines Gels, eines Schaums, einer Tinktur, einer Lösung oder in Form von Augentropfen.

6. Pharmazeutisches Präparat gemäss Anspruch 5 in Form von Augentropfen.

7. Verbindung der Formel I

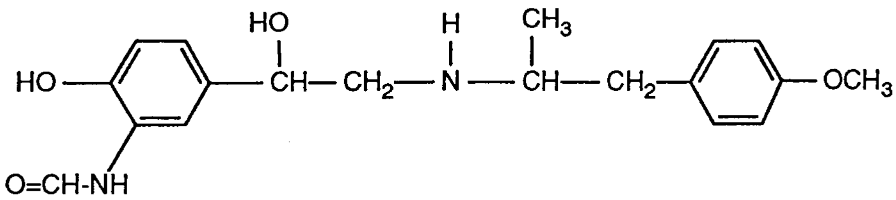

(I)

oder eines pharmazeutisch verwendbaren Salzes davon zur topischen, d.h. dermalen, Behandlung entzündlicher Hauterkrankungen.

**8.** Verwendung einer Verbindung der Formel I

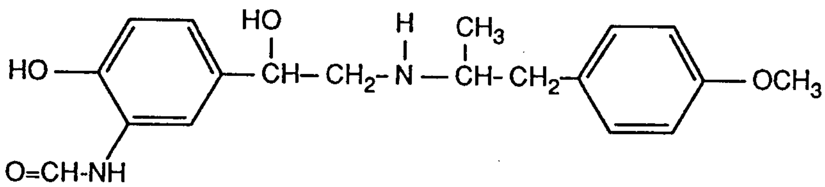

(I)

oder eines pharmazeutisch verwendbaren Salzes davon zur Herstellung eines pharmazeutischen Präparats zur dermalen Behandlung entzündlicher Hauterkrankungen.

## Claims

**1.** A pharmaceutical preparation comprising a compound of the formula I

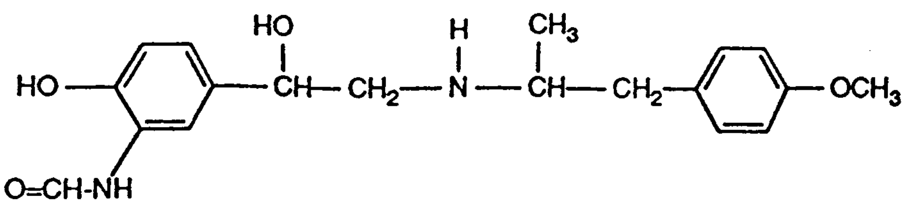

(I),

or a pharmaceutically acceptable salt thereof, together with customary pharmaceutical adjuncts, characterised in that it is intended exclusively for topical, i.e. dermal, application.

**2.** A pharmaceutical preparation according to claim 1, comprising as active ingredient the semifumarate of a compound of the formula I.

**3.** A pharmaceutical preparation according to either claim 1 or claim 2, comprising an active ingredient content of from approximately 0.00001 to approximately 1 % by weight.

**4.** A pharmaceutical preparation according to claim 3, comprising an active ingredient content of from approximately 0.00005 to approximately 0.5 % by weight.

**5.** A pharmaceutical preparation according to any one of claims 1 to 4 in the form of a cream, an ointment, a fatty ointment, a paste, a gel, a foam, a tincture or a solution or in the form of eyedrops.

**6.** A pharmaceutical preparation according to claim 5 in the form of eyedrops.

**7.** A compound of the formula I

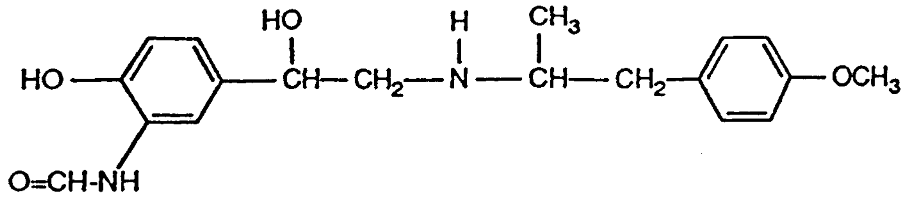

(I),

or a pharmaceutically acceptable salt thereof, for the topical, i.e. dermal, treatment of inflammatory skin diseases.

**8.** The use of a compound of the formula I

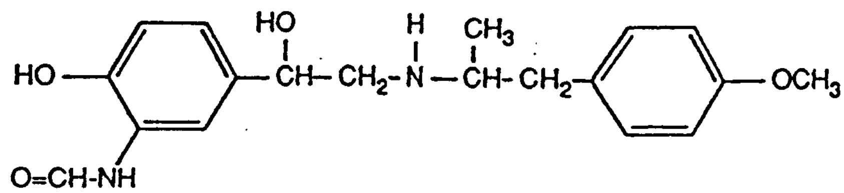

(I),

or a pharmaceutically acceptable salt thereof, for the manufacture of a pharmaceutical preparation for the dermal treatment of inflammatory skin diseases.

## Revendications

**1.** Composition pharmaceutique contenant un composé de formule I

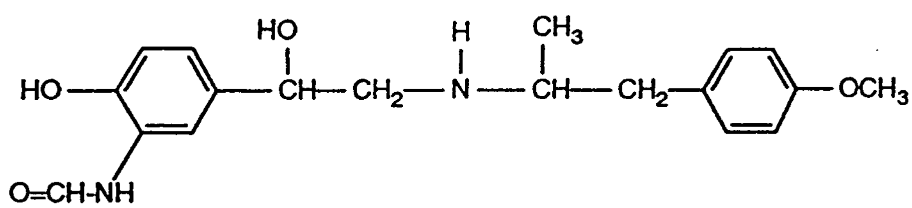

(I)

ou un sel acceptable pour l'usage pharmaceutique d'un tel composé, avec des produits auxiliaires pharmaceutiques usuels, caractérisée en ce qu'elle est prévue exclusivement pour l'application topique, c'est-à-dire dermique.

**2.** Composition pharmaceutique selon la revendication 1, contenant en tant que substance active l'hémifumarate d'un composé de formule I.

**3.** Composition pharmaceutique selon la revendication 1 ou 2, contenant la substance active en proportion d'environ 0,00001 à 1 % en poids.

**4.** Composition pharmaceutique selon la revendication 3, contenant la substance active en proportion d'environ 0,00005 à 0,5 % en poids.

**5.** Composition pharmaceutique selon l'une des revendications 1 à 4, sous forme de crème, de pommade, de pommade grasse, de pâte, de gel, de mousse, de teinture, de solution ou de gouttes oculaires.

**6.** Composition pharmaceutique selon revendication 5, sous la forme de gouttes oculaires.

**7.** Composé de formule I

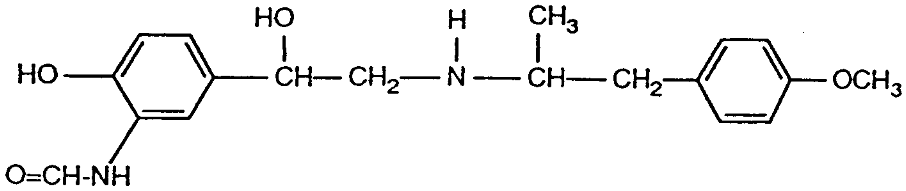

(I)

ou l'un de ses sels acceptables pour l'usage pharmaceutique, pour le traitement topique, c'est-à-dire dermique, des maladies inflammatoires de la peau.

**8.** Utilisation d'un composé de formule I

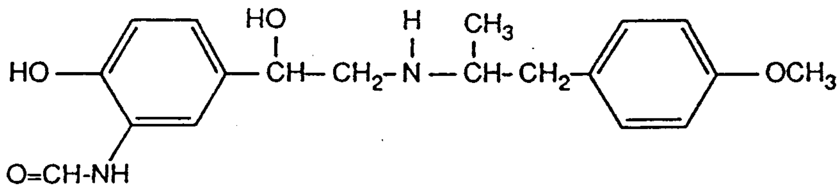

(I)

ou d'un sel acceptable pour l'usage pharmaceutique d'un tel composé, pour la préparation d'une composition pharmaceutique pour le traitement dermique des maladies inflammatoires de la peau.